Europäisches Patentamt

European Patent Office

Office européen des brevets

(11) Publication number: **0 335 597 B1**

# EUROPEAN PATENT SPECIFICATION

(45) Date of publication of patent specification: 26.05.93 (51) Int. Cl.5: **A61K 37/50**, A61K 9/50

(21) Application number: **89302914.0**

(22) Date of filing: **23.03.89**

(54) **Use of a polypeptide in the preparation of a composition for the treatment of pancreatitis.**

(30) Priority: **28.03.88 JP 73942/88**

(43) Date of publication of application:
**04.10.89 Bulletin 89/40**

(45) Publication of the grant of the patent:
**26.05.93 Bulletin 93/21**

(84) Designated Contracting States:
**DE ES FR GB IT**

(56) References cited:
EP-A- 0 225 130
WO-A- 87/01387

**DIGESTIVE DESEASES AND SCIENCES, vol. 32, no. 10, October 1987, page 1191; J.R. WISNER et al.: "The effects of a polyethylene glycol:superoxide dismutase adduct on cerulein-induced acute pancreatitis in the rat"**

**BIOLOGICAL ABSTRACTS, vol. 85, 1988, abstract no. 110183, Biological Abstracts Inc., Philadelphia, PA, US; A.S. GODDIO: "Superoxide dismutase: Animal experimentation and applications in plastic surgery"**

**Y. NIWA et al, Free Red. Res. Comms., 1,**

**(1985), pp. 137-153**

(73) Proprietor: **ASAHI KASEI KOGYO KABUSHIKI KAISHA**
**2-6, Dojimahama 1-chome Kita-ku Osaka(JP)**

Proprietor: **Tosoh Corporation**
**4560, Kaisei-cho**
**Shinnanyo-shi, Yamaguchi-ken(JP)**

(72) Inventor: **Nakae, Dai**
**4-500-34 Higashi-ikoma**
**Ikoma-shi Nara-ken(JP)**
Inventor: **Konishi, Yoichi**
**No. 215 Jyoroku**
**Sakai-shi Osaka-fu(JP)**
Inventor: **Nagata, Yoshihiko**
**1-9-1-203 Minamidai**
**Sagamihara-shi Kanagawa-ken(JP)**
Inventor: **Kobayashi, Youshiro**
**719-5 Moriki Ohito-cho**
**Tagata-gun Shizuoka-ken(JP)**

(74) Representative: **Woods, Geoffrey Corlett et al**
**J.A. KEMP & CO. 14 South Square Gray's Inn London WC1R 5EU (GB)**

**Description**

This invention relates to the use of human Cu,Zn‒SOD or a mutein thereof in the preparation of a composition for the treatment of pancreatitis.

The pancreas comprises exocrine gland tissue, which is 90% of the organ, and endocrine tissue consisting of Langerhans islets. Enzymes of pancreatic origin are known, for example protease such as trypsin, chymotrypsin, elastase or leucine aminopeptidase, $\alpha$‒amylase which hydrolyses $\alpha$‒1,4‒glycoside bonds (hereinafter referred to as amylase), lipase which hydrolyses neutral fat to monoglyceride and fatty acid, cholesterol esterase and phospholipase. Insulin is secreted from B‒cells in the Langerhans islets and pancreatic glucose is secreted from pancreatic A cells. The pancreas also secretes various peptide hormones and is controlled by other peptide hormones.

Pancreatitis is classified in 4 types according to Marseilles' classification, namely acute pancreatitis and chronic pancreatitis each of which is separated into two types, i.e. acute pancreatitis, recurrent acute pancreatitis, chronic pancreatitis and chronic recurrent pancreatitis.

The main causes of pancreatitis are cholelithiasis and continued over‒drinking of alcohol. Other types of pancreatitis are, for example, postoperative pancreatitis, parotid gland pancreatitis, parathyroid pan‒creatitis or drug induced pancreatitis. Steroid, chlorothiazid or tetracycline administered induced pancreatitis is also known.

Clinical diagnosis of pancreatitis is carried out by assaying for increased serum and urinary lipase or amylase activity.

Among polypeptides having superoxide dismutase activity, a polypeptide containing copper and zinc (Cu,Zn‒SOD), a polypeptide containing manganese (Mn‒SOD) and a polypeptide containing iron (Fe‒SOD) have been known to be widely distributed in animals, plant and microorganisms [Advances in Enzymology, 41: 35 ‒ 97 (1974)]. Superoxide dismutase catalyses the reaction shown below, on a superoxide anion radical ($O_2^-$) to generate hydrogen peroxide ($H_2O_2$) and molecular oxygen.

$$2O_2^- + 2H^+ \rightarrow O_2 + H_2O_2$$

The polypeptide having superoxide dismutase (hereinafter designated as SOD) activity is useful to scavenge superoxide anion radicals, which are detrimental to cells and tissues. It has been used in the treatment of rheumatic arthritis [Current Therapeutic Res., 20: 62 ‒ 69 (1976)] and as an anti‒inflammatory agent for the skin [PCT. No. 62‒501072].

No reports are known that a polypeptide having SOD activity per se has a curative effect against pancreatitis.

We have found that a polypeptide having SOD activity, when administered per se to animals with pancreatitis induced by drug injection, gave no unexpected decrease in the level of serum lipase and amylase activity, a marker of pancreatitis.

We have, however, found that a liposomal preparation of a polypeptide having SOD activity admin‒istered to animals suffering from pancreatitis unexpectedly reduced the level of serum lipase and amylase activities effectively.

The present invention provides the use of liposomes comprising a polypeptide having superoxide dismutase activity in the preparation of a medicament for the treatment of pancreatitis, wherein said polypeptide is human Cu,Zn‒superoxide dismutase or a mutein thereof wherein cysteine at position 111 has been replaced by serine.

An extracted and purified polypeptide of natural origin, a polypeptide prepared by chemical synthesis or genetic engineering means, or a mutein [Glossary of Genetics and Cytogenetics, 4th Ed., p.381 (1976)] which is a partially modified peptide with SOD activity provided by chemical synthesis or genetic engineering, is preferably used. The polypeptide contains copper and zinc, is a dimer and has a M.W. of approximately 32000. It is widely distributed in humans.

The human Cu,Zn‒SOD polypeptide is constituted by at least 153 amino acids, and can be obtained by the following procedures.

RNA is isolated from human liver tissue, further poly(A) + RNA is isolated, then c‒DNA is synthesized therefrom by an ordinary genetic engineering technique. The c‒DNA is inserted in a plasmid to prepare an expression vector, which is then transformed into a microorganism such as E. coli or yeast. These microorganisms are cultured to produce a dimer polypeptide having SOD activity. These genetic engineer‒ing techniques are disclosed in Japanese Patent Unexamined Publication No. 60‒137286, ibid. No. 61‒111690, ibid. No. 61‒139390, ibid. No. 62‒115277 and ibid. No. 62‒130689.

The polypeptide may also be a mutein polypeptide in which cystein at position 111 has been replaced by serine maintaining the SOD activity. For example the expression vector obtained hereinbefore is subjected to site−specific recombination. Namely a closed circular plasmid vector containing DNA corresponding to a polypeptide having natural human type SOD activity is treated with restriction enzyme Bam HI in the presence of ethidium bromide under dark conditions to prepare open circular DNA, which is treated with exonuclease II. Then the treated DNA is annealed with another single−strand DNA which contains a base sequence coding an amino acid to be modified, and the mixture is treated with DNA polymerase and $T_4$ ligase in the presence of dNTP to bind synthetic DNA into plasmid vector [Experimental Manipulation of Gene Expression, 291−303 (1983), Academic Press. ]. The thus obtained vector in which the expected specific site of the DNA sequence is modified is tranferred by a common genetic engineering method into microorganisms to express the dimer polypeptide, which is isolated by a generally known method. These genetic engineering methods are disclosed in Japanese Patent Unexamined Publication No. 62−130684. In a mutein polypeptide having natural human type SOD activity, i.e. a dimer Cu,Zn−SOD consisting of 153 amino acids with N−terminal alanine at position−1, cysteine at position−6 and/or at position−111, the cysteine at position−111 is converted to serine and the N−terminal is hydrogen or an acetylated group. The cysteine at position−6 is cysteine per se or is converted to a neutral amino acid such as serine, alanine or threonine.

These polypeptides can also be prepared in the form of a non−toxic soluble salt.

A liposome is a small vesicle consisting of lipid bilayer containing a water phase in an inner crust.

A liposomal preparation which contains the polypeptide having SOD activity can be produced by known method, for example that disclosed in Biochim. Biophys. Acta, 135 : 624 − 638 (1967), ibid., 443 : 629 − 643 (1976), ibid., 601 : 559(1980), ibid., 542 : 137(1978), ibid ., 649 : 129(1981), ibid., 394 : 483(1975), ibid., 646 : 1(1 981), ibid., 728 : 339(1983), ibid., 770 : 109(1984) ibid., 817 : 193(1985), U.S. Patent 4,235,871, Biochemistry, 8 : 34 4(1969), Proc. Natl. Acad. Sci. U.S.A., 76 : 145(1979), ibid ., 75 : 4194(1978) Ann. Rev. Biophys. Bioeng., 9 : 467(198 0) or J. Am. Chem. Soc., 106 : 1897(1984).

Many kind of types and forms of liposomes, for examples a small−unilamellar vehicle (SUV), large−unilamellar vehicle (LUV), oligolamellar vehicle (REV), multilamellar vehicle (MLV) and stable plurilamellar vehicle (SPLV), can be used.

The compositions of the liposome itself is substantially phospholipid, stabilizing agent and electric charging agent. Examples are natural or synthetic phospholipids preferably natural phosphatidylcholine such as egg−lecithin and soybean lecithin or their hydrogenation products, saturated synthetic lecithin such as dimyristoylphosphatidylcholine, dipalmitoyl phosphatidylcholine and distearoyl phosphatidylcholine, or unsaturated synthetic lecithin such as dioleoyl phosphatidylcholine and dilinoleoyl phosphatidylcholine, or a mixture thereof, or another phospholipid such as sphingomyelin, phosphatidylethanolamine, phosphatidylglycerol, phosphatidylserine and ceramide phosphorylethanolamine, a lipid of a stabilizing agent such as cholesterol, a liposome cationic electric charging agent such as cetylamine and stearylamine, and anionic electric charging agent such as diacetylphospahte, phosphatidylserine and phosphatidic acid. In general, the ratios of phospholipid : stabilizing agent : electric charging agent are 100 to 50 weight parts : 0 to 30 weight parts : 0 to 20 weight parts, preferably 75 to 60 : 20 to 15 : 15 to 10 weight parts. Another preferred example is a lipid composition of phospholipid : electric charging agent of 90 to 85 : 10 to 15 weight parts. If required, a lipid stabilizing agent such as tocopherol or $\beta$−carotene can be added.

A lipid composition prepared as above is dissolved in a single or mixed solvent, for example a volatile soluble medium such as diethyl ether, chloroform, methylene chloride, ethylacetate, petroleum ether or methanol.

The above lipid composition solution is poured into a round flask and the solvent is removed, such as by a rotary evaporator, to form a thin layer of lipid composition on the inner side of the flask. If necessary the operation of adding and removing solvent is repeated and finally the medium should be removed completely in vacuo.

A solution of the polypeptide (0.01 to 100 mg/mℓ) having SOD activity dissolved in distilled water or buffer solution for injection is added to the lipid composition in the flask at a ratio of 5 to 200 weight parts of the solution to one weight part of the lipid composition. The amount of polypeptide can be 0.01 to 0.5 weight parts per one weight part of lipid composition. The mixture is, if required, warmed to from 35 to 60 °C in a water−bath, stirred and suspended to obtain a liposome solution. The suspension is treated with ultrasonication at 10 KC for 1 to 30 minutes to prepare liposomes of uniform size. By ultrasonicaton, liposomes containing the polypeptide having SOD activity mainly consisting of MLV 50 to 550 nm in diameter can be prepared.

The thus prepared liposomes can be isolated by centrifugation at 10,000 to 20,000 G for 15 to 60 minutes. The liposomes can also be treated by ultracentrifugation for concentration or membrane filtration for sterilization. Injectable pharmaceutical preparations can be prepared by suspending the liposomes in a liquid for injection to produce a suspension, or by adding a lyophilization stabilizing agent such as glucose, sucrose, mannitol or sorbitol at a concentration of from 0.5 to 10 % to the liposome solution and lyophilizing to produce a suspension for intramuscular, subcutaneous or intravenous injection before use. The concentration of the polypeptide having SOD activity is preferably 0.01 to 100 mg per 1 ml of medicament, for one to five times administration in a day with 0.1 to 2 mℓ in one injection. To the liposome preparation can optionally be added an osmotic pressure regulating agent, antiseptics or a pH controlling agent.

Suppositories of the liposome preparation can also be prepared by mixing a liposome with one or more suppository oleaginous bases, for example a fat or oil such as olive oil, peanut oil, sesame oil, soybean oil, rapeseed oil, camellia oil, cacao butter, lard, lanolin and beef tallow, hydrogenated or acetylated derivatives thereof, and emulsified fat and oil with a surface active agent, or furthermore adding one or more aqueous bases such as glycerogelatin and propyleneglycol thereto to prepare a 1 to 2 g per unit suppository formulation.

Granules, capsules or a syrup formulation can also be prepared by adding ingredients such as fillers, lubricants, sweeteners, colours or aromas.

The thus prepared liposomes containing the polypeptide having SOD activity are, as illustrated in the following Examples, useful for therapeutic drug compositions for pancreatitis, which can reduce the serum level of lipase and amylase activity on drug induced pancreatitis cell injury.

The following examples further illustrate the present invention.

EXAMPLE 1

Production of liposomes containing polypeptide having SOD activity:

A chloroform solution containing 7 mM L−α−dipalmitoyl phosphatidylcholine (hereinafter designated L−α−DPPC), 1 mM colesterol and 2 mM stearylamine was prepared. The solvent was removed in vacuo from a 250 ml sample of the above chloroform solution containing the three above lipids in an amount of 2.5 mM. The mixture was then treated in vacuo of a few mm Hg by a oil rotary vacuum pump for 3 hours to remove the solvent further and to prepare a thin layer lipid membrane. A phosphate buffer solution (4mM, pH 7.2, 100mℓ) of a polypeptide having human type Cu,Zn−SOD activity (hereinafter designated SOD−1, 100mg, specific activity 3200 u/mg), wherein cysteine at position−111 was site−specifically replaced by serine, which was obtained by culturing E. Coli DH1 carrying plasmid pSOD14 prepared according to the method disclosed in JP−A−62−130684, was added thereto and heated at 40 °C for 6 minutes to disperse the lipid, and then treated by ultrasonication.

The thus obtained suspension was allowed to stand for 3 hours at room temperature and thereafter overnight at 15 °C. No encapsulated free SOD−1 was removed by centrifugation at 12000 rpm, at 4 °C for 60 minutes.

The prepared liposomes containing SOD−1(hereinafter designated as L−SOD−1) had the following properties.

| | |
|---|---|
| Particle size | : 205 − 550 nm; average diameter 340 − 370nm. |
| Encapsulation ratio of SOD−1 | : 87 % |
| Specific activity of SOD−1 | : 3200 U/mg |
| Recovery of SOD−1 | : 84 % |

The above were measured according to the following method and equations:

$$= \frac{\text{SOD total activity-SOD supernatant activity}}{\text{SOD total activity}} \times 100$$

The SOD total activity was measured by adding 1 % Triton® X−100 to a sample and incubating at 37°C for 30 minutes.

The SOD supernatant activity was measured after centrifuging the sample at 14,000 rpm (approx. 20000 G) for 30 minutes at 4 °C.

4

The SOD activity was assayed accroding to a NBT-method (Anal. Biochem., 44 : 276 - 287 (1971).

$$\text{Specific activity(U/mg)} = \frac{\text{SOD-total activity}}{\text{Total protein (weight)}}$$

The amount of protein was measured according to the method of Lowry et al.

$$\text{Recovery (\%)} = \frac{\text{SOD in liposome}}{\text{Total SOD used}} \times 100$$

EXAMPLE 2

The SOD-1 in example 1 was replaced by Cu,Zn-SOD extracted from human erthrocytes (hereinafter designated SOD-2) [Development in Biochemistry, Biological and Clinical Aspects of Superoxide and Superoxide Dismutase 11B : 348 (1980)] to obtain liposomes containing SOD-2 (hereinafter designated as L-SOD-2).

EXPERIMENT 1

The effects of L-SOD-1 and L-SOD-2 on drug-induced pancreatitis using 4-hydroxyaminoquinoline-1-oxide (hereinafter designated as 4-HAQO) were determined as follows:

Female Wister rats were divided into 5 groups each of which consisted of 5 to 7 rats.
Each group was separated as follows.
Group I:　　Control, non treated group.
Group II:　　4-HAQO dissolved in distilled water for injection was administered intravenously at 20 mg/kg body weight.
Group III:　　4-HAQO dissolved in distilled water for injection was administered intravenously at 20 mg/kg body weight, and simultaneously L-SOD-1 suspended in distilled water for injection was administered intraperitoneally at 16 mg (weight of SOD-1)/kg body weight. After 12 hours the same amount of L-SOD-1 (16 mg equivalent SOD-1) was again administered intraperitoneally.
Group IV:　　4-HAQO dissolved in distilled water for injection was administered intravenously at 20 mg/kg body weight, and simultaneously L-SOD-2 suspended in distilled water for injection was administered intraperitoneally at 16 mg (weight of SOD-2)/kg body weight. After 12 hours the same amount of L-SOD-2 (16 mg equivalent SOD-2) was again administered intraperitoneally.
Group V:　　4-HAQO dissolved in distilled water for injection was administered intravenously at 20 mg/kg body weight, and simultaneously non-liposomal SOD-1 (free SOD-1) dissolved in distilled water for injection was administered intraperitoneally at 16 mg/kg body weight. After 12 hours the same amount of free SOD-1 (16 mg/kg) was again administered intraperitoneally.
All the animals were anesthesized with ether after 24 hours from the 4-HAQO administration, and blood was collected from the abdominal aorta. The animals were then autopsied.
The extent of the pancreatitis cell damage was assessed by assaying the activities of lipase (Autopack Lip, Tradename Boehringer Mannheim Co.) [Ziegenherm, J. et al., Clin. Chem., 25 : 1067(1979), and Verduin, C.A., et al., Clin. Chim. Acta, 46 : 11(1973)], and amylase [Clinimate AMY-C, Tradename, Daiichi Kagaku Co.) [Okawa, J. "Clinical Test Technique", vol. 12, p.225(1984), and Kanai, I, Ed. "Clinical Laboratory Tests Commentary", 29th Ed. p.772 (1983)].

The results are shown in Table 1:

Table 1

| Group | I | II | III | IV | V |
|---|---|---|---|---|---|
| No.of animals | | | | | |
| at start | 5 | 7 | 7 | 7 | 6 |
| at biopsy | 5 | 7 | 7 | 7 | 6 |
| lipase ( 10U/l) | 23[1] ±10 | 147 ±56 | 66* ± 20 | 56* ±18 | 131 ±61 |
| Amylase (100 s.u.) | 31 ±4 | 212 ±44 | 178 ±45 | 175 ±42 | 250 ±34 |

1) Mean ± SD
*) significant difference from II Group and V Group results at $P < 0.05$

As illustrated above, increased levels of serum lipase and amylase caused by 4−HAQO induced pancreatic cell damage of pancreatitis, were protected significantly by administration of L−SOD−1 and L−SOD−2. On the contrary no improvement of clinical examination on drug−induced pancreatitis was observed when free SOD−1 was administered.

## Claims

1. Use of liposomes comprising a polypeptide having superoxide dismutase activity in the preparation of a medicament for the treatment of pancreatitis, wherein said polypeptide is human Cu,Zn−superoxide dismutase or a mutein thereof wherein cysteine at position 111 has been replaced by serine.

2. Use according to claim 1 wherein said polypeptide is a natural polypeptide.

3. Use according to claim 1 wherein said polypeptide is a mutein polypeptide.

4. Use according to any one of claims 1 to 3 wherein said polypeptide is present in an amount of from 0.01 to 100 mg per 1 ml of medicament.

## Patentansprüche

1. Verwendung von ein Polypeptid mit Superoxiddismutase−Aktivität umfassenden Liposomen bei der Herstellung eines Medikaments zur Behandlung von Pankreatitis, worin das Polypeptid Human−Cu,Zn−Superoxiddismutase oder ein Mutein davon ist, worin Cystein in Position 111 durch Serin ersetzt wurde.

2. Verwendung nach Anspruch 1, worin das Polypeptid ein natürliches Polypeptid ist.

3. Verwendung nach Anspruch 1, worin das Polypeptid ein Mutein−Polypeptid ist.

4. Verwendung nach irgendeinem der Ansprüche 1 bis 3, worin das Polypeptid in einer Menge von 0,01 bis 100 mg pro 1 ml Medikament zugegen ist.

## Revendications

1. Utilisation de liposomes comprenant un polypeptide ayant une activité de superoxyde dismutase, dans la préparation d'un médicament pour le traitement de la pancréatite, où ledit polypeptide est Cu,Zn−superoxyde de dismutase d'être humain ou bien sa mutéine où la cystéine à la position 111 a été remplacée par la sérine.

2. Utilisation selon la revendication 1, où ledit polypeptide est un polypeptide naturel.

**3.** Utilisation selon la revendication 1, où ledit polypeptide est un polypeptide de mutéine.

**4.** Utilisation selon l'une quelconque des revendications 1 à 3, où ledit polypeptide est présent en une quantité de 0,01 à 100 mg pour 1 ml du médicament.